# EUROPEAN PATENT SPECIFICATION

(11) **EP 3 232 777 B1**
(45) Date of publication and mention of the grant of the patent: **17.01.2024**
(21) Application number: 15813848.7
(22) Date of filing: 18.12.2015
(51) Int. Cl.: A01N 1/02, A61N 7/00, A61F 7/10

(54) **PRESERVATION AND TRANSPORT OF AN EX VIVO BIOLOGICAL SAMPLE COMPRISING ULTRASOUND APPLICATION**
KONSERVIERUNG UND TRANSPORT EINER BIOLOGISCHEN EX-VIVO-PROBE UNTER VERWENDUNG VON ULTRASCHALL
TRANSPORT ET CONSERVATION D'UN ÉCHANTILLON BIOLOGIQUE EX-VIVO UTILISANT DES ULTRASONS

(30) Priority: 19.12.2014 EP 14382548
(43) Date of publication of application: 25.10.2017
(73) Proprietor: FUNDACIÓ DE RECERCA CLÍNIC BARCELONA-INSTITUT, 08036, Barcelona (ES)
(72) Inventor: PERALTA, Carmen, 08036 Barcelona (ES)
(74) Representative: Hoffmann Eitle
(86) International application number: PCT/EP2015/080688
(87) International publication number: WO 2016/097390

(56) References cited:
- WO-A2-2007/143715
- WO-A2-2011/071727
- JP-A- H01 308 201
- US-A1- 2005 147 958
- US-A1- 2008 145 919
- US-B1- 6 673 594

## Description

### Field of the invention

The invention relates to a device for transport and preservation of an *ex vivo* biological sample comprising a chamber for containing the biological sample, delimitated by walls made of a thermal insulating material and cooling means for keeping the temperature inside the chamber below the temperature outside the device, as well as to a corresponding method for preservation of the biological sample.

The invention also relates to the use of the device according to the invention for transport and preservation of an *ex vivo* biological sample with a view to subsequent transplant in a living human being or animal, or for subsequent laboratory research.

### State of the art

In the invention, the concept of *"ex vivo* biological sample" relates to an organ or tissue that can be transported for subsequent transplant in a living human being or animal, or for subsequent laboratory research.

For example, it is well known in the field of transplants that ischemia/reperfusion (I/R) syndrome is one of the main causes of both primary graft dysfunction (PGD) and primary graft failure (PGF), another transplantation being necessary in the latter case. Despite the advances achieved in surgical techniques in the past few decades, the injury the graft sustains during the period in which the donor organ is transported until it is implanted in the recipient (cold ischemia time) is still a major socially and clinically relevant problem yet to be solved. The incidence of PGF is 5-10% and the incidence of PGD is 20-30% among transplanted organs. This all negatively results in a poor quality of life for the patient, the need for transplanting again, and more problems in the availability of organs for transplant.

There are different methods for storing a graft from the time it is removed from the donor until it is implanted in the recipient. In the case of dynamic normothermic preservation, *in vivo* conditions of the graft are imitated to the greatest extent possible throughout the entire preservation period. To that end, hypothermia is avoided, and the entrance of oxygen into the graft to prevent hypoxia and a reservoir for the exit of waste product are provided.

In static hypothermic preservation, which is the conventional preservation method, before the donor graft is transplanted in a recipient, the donor's organ or organs and tissues are subjected to an inherent ischemia time. Under such conditions, the method used for preserving the organ and tissues must comply with the following requirements: removing blood from the graft and rapidly cooling the graft by means of perfusion of the graft with the solution at 4°C, covering the graft with ice to assure cold conditions (2-6°C) and ischemic conditions. As a result, the injury the graft sustains during cold ischemia, i.e., due to a lack of blood supply and oxygen delivery under cold conditions, is reduced.

From the time the organ is removed from the donor, the graft is placed in a cooler, immersed in the preservation solution, and it is usually kept at 4°C until it is implanted in the recipient. The solution most widely used in the art is the University of Wisconsin solution, hereinafter UW solution.

The injury the graft sustains during transport in the cooler until it is implanted in the recipient (cold ischemia time, which is not usually less than 6 hours) is a key factor. This factor is responsible for PGD and PGF and negatively affects post-operative results, the quality of life and survival of the patient. It is known that the longer the time the organs remain in the cooler, the lower the viability of the graft that will be transplanted. The injury grafts sustain during the cold ischemia stage is the primary reason that a considerable number of organs (35%) cannot be considered suitable for transplant given their pathological conditions (kidney grafts from elderly, diabetic and/or hypertensive donors, fatty liver grafts, etc...). Such organs are extremely vulnerable to the injury they sustain during transport, before being implanted in the recipient, and have a higher risk of experiencing PGD and PGF. They are therefore not viable for transplant. These observations indicate that there is a need to find alternatives for reducing the injury grafts sustain during cold ischemia before being implanted in the recipient.

Many of the components present in preservation fluids, the objective of which is to protect the graft under cold ischemia conditions in the cooler during the transport thereof until it is implanted in the recipient, do not enter the graft and if they do, they do not reach the site of action at optimal concentrations in order to protect it. In fact, it has been found that when some of the components are removed from such preservation fluids, postoperative and post-transplant results are the same as if they were not removed.

Patent document US 6 673 594 B1 describes an organ perfusion apparatus to sustain, monitor and/or restore organ viability by perfusing the organ with an oxygenated medical fluid at hypothermic temperature and/or normothermic temperatures. This apparatus is delimitated by walls made of a thermal insulating material, and may further comprise cooling means and an auxiliary container with one or two lids for containing an organ to be perfused. This auxiliary container can be formed with a fluid tight lower portion in which the effluent medical fluid can collect when the organ is perfused.

Patent document WO 2007/143715 A2 relates to applying ultrasound to allow delivering oxygen to wounds and to aid wound healing. By way of example, the patent document very briefly discloses a device for preserving organs after they have been removed from the donor. To that end, the organ is placed in a vessel which is supplied with a gas or liquid supersaturated with oxygen. It is a dynamic preservation system because the oxygen or liquid solutions saturated with oxygen contained therein gradually enter at controlled flow from an oxygen reservoir into the vessel, and waste products are gradually removed. The organ vessel is placed inside a semi-rigid container. The outer container further comprises an ultrasound system for directing ultrasound on the container. There are no means for transmitting ultrasound between the inner vessel and the outer container, so the system cannot work as well as if the ultrasound was applied directly on a wound for the purpose of wound healing.

Studies indicating that application of ultrasound aids in delivering drugs and substances through tissues, such as the skin, and into the bloodstream, are also known. Nevertheless, in said studies ultrasound is applied *in vivo,* i.e., in the presence of blood flow. Patent document US 4,767,402 A, relating to the field of transdermal drug delivery by means of applying ultrasound, also relates to the risks associated with the use thereof. The patent document particularly advises against the prolonged use of ultrasound due to the risks associated with the increase in skin temperature and the possible damage this technique can cause. The same patent document also discloses that ultrasound is poorly transmitted through air and that the preferred application thereof therefore requires a liquid medium. As a result, applying ultrasound for organ and tissue preservation is not advisable due to the mentioned factors of: local heating of the tissue, difficulty in penetrating considerable tissue or organ thicknesses, and transmission difficulty in gaseous media.

Patent document US 5,267,985 A discloses a method and apparatus for enhancing the diffusion of a substance to a local area of a material or tissue by means of ultrasound at two or more distinct frequencies. This patent document also discloses that the local temperature increase when ultrasound is applied is beneficial for aiding in increasing penetration of the substances to be diffused.

The article (Sen Wang et al., Medial hypothesis, 74, 2010; 147-149) suggests applying ultrasound at a medium intensity (0.3-1.2 W/cm²) *in vivo* in order to damage a specific area of the donor organ such that only stem cells are activated and the organ can be regenerated, forming a hybrid organ that can be more compatible with the recipient.

Patent document WO 2005/013799 A1 discloses a method based on applying ultrasound *in vivo* during reperfusion, i.e., blood supply enters the tissue, after an ischemia time. Ultrasound is only applied for a short time span (not more than 15 minutes) to favor the entrance of blood and oxygen into a tissue and to reduce microcirculation disorders caused by reperfusion.

Therefore, it can be inferred from the art that applying ultrasound generates heat in both aqueous media and tissues. Ultrasound applied for only between 1-10 minutes (times used in most medical applications) and at frequencies and intensities that are usual in the art alone causes the heating of tissues, which is interpreted as a negative factor for proper storage of an *ex vivo* biological sample. When the temperature exceeds 38ºC, exposure to ultrasound is usually interrupted because it damages tissues. For example, patent document US 5,267,985 discloses the possibility of using ultrasound for 15-30 minutes to achieve tissue penetration between 1 and 2 cm.

In fact, ultrasound has often been applied in clinical practice precisely as therapy to cause the heating of tissue. Besides the heating caused by ultrasound alone, ceramic transducers tend to heat up with vibration, even further increasing the thermal effect on tissue (Watson T *et al.,* 2008, 48: 321-329; Baker KG, *et al.,* 2001; 81: 1351-1358; Legay M *et al.;* 2011, ID670108; 1-17).

JPH 01-308201 A discloses an organ preservation apparatus comprising an insulated chamber, an organ housing, a perfusion circuit, oxygenation means, cooling means to maintain the temperature of the perfusate at about 7 °C, and an ultrasound system to generate a tomogram of the organ.

### Summary of the invention

The invention is defined in the appended claims.

It is an object of the invention to provide a device for transport and preservation of an *ex vivo* biological sample and a method of the type indicated above that reduces injury to the biological sample, and accordingly increases viability thereof in comparison to what could be obtained with known devices. The invention also considers, in the case of transplants, the problem of improving the quality of life of transplant patients, and in the worst cases, of significantly reducing the need for another transplant.

This objective is achieved by means of a device for *ex vivo* transport of the type indicated above, which further comprises at least an ultrasound system suitable for generating and applying ultrasound on said biological sample. Applying ultrasound combined with cooling the inner chamber can therefore be carried out such that local heating of the biological sample, and the subsequent increase in temperature that would cause damage therein, are prevented. The device also comprises a closable auxiliary container containing a preservation solution, without external oxygen delivery and for containing said biological sample immersed in said preservation solution.

Therefore, contrary to what was expected, it has been verified in the invention that applying ultrasound, which is applied such that it does not generate local heating in the sample, does not negatively affect the positive effect of cooling the biological sample, and accordingly does not damage it either. Furthermore, however, it has surprisingly been verified that a very significant improvement is achieved with ultrasound, so much so that as will be seen later in the description, a synergistic effect between applying cold conditions inside the chamber of the device and applying ultrasound could be confirmed. To that end, there are many possible configurations, such as applying ultrasound close to the biological sample at a low intensity, or arranging the transducers away from the sample at higher intensities and in contact with a preservation fluid containing the sample.

Furthermore, also contrary to what was expected, problems with the duration of applying ultrasound were not verified in the invention. In other words, the need to keep the sample under cold ischemia conditions for several hours is known in organ transport. According to the invention, ultrasound can be continuously applied at suitable intensities, and despite what would be expected, it does not damage the biological sample either.

Therefore, by applying ultrasound, and preventing local heating of the sample, a significant reduction of cell damage has been verified. Furthermore, based on the assays conducted, it has been verified that protection of the organ against injury induced by cold ischemia by simultaneously applying both treatments is unexpectedly better than the sum of all the protection obtained through the treatments separately.

Furthermore, the invention covers a series of preferred features which are the object of dependent claims and the usefulness of which will be highlighted below in the detailed description of an embodiment of the invention.

In a preferred embodiment, it has been verified that said ultrasound system is suitable for emitting said ultrasound at a frequency comprised between 25 kHz and 1 MHz and a sound intensity comprised between 0.01 and 2 W/cm², and preferably between 0.02 and 1 W/cm², and particularly preferably between 0.02 and 0.1 W/cm². These frequency and intensity ranges have been verified to be particularly beneficial in reducing cell damage. Particularly at a lower intensity, better results are obtained and less cooling of the sample and/or separation of the transducers with respect to the biological sample is necessary, which allows the device to be more compact.

It can also preferably be envisaged that the ultrasound is applied in a continuous manner or alternatively in a pulsed manner. Furthermore, the invention does not rule out simultaneously applying ultrasound having different frequencies by means of the corresponding individual control of each of the transducers of the device.

It has also been verified that the lower the temperature, the greater the synergistic effect obtained with the ultrasound. Therefore, in a particularly preferred manner, the cooling means are suitable for keeping the temperature inside said chamber between 0 and 15ºC, and preferably between 2 and 10ºC, and particularly preferably between 2 and 6ºC. So much so that as has been verified, the combination of cold conditions and ultrasound together provides better results than what would be expected in the best case of the sum of the effects of cold conditions and ultrasound separately.

The device according to the invention comprises a sheet-like support in said chamber suitable for supporting the biological sample, said sheet-like support being able to vibrate freely when said ultrasound is applied, and the ultrasound system comprises at least one transducer mounted on at least one of the walls of said sheet-like support. As a result, the damping effect of the support is minimized and ultrasound is introduced in the biological sample in a more efficient manner, improving the effect thereof in bulkier biological samples provided that excessive local heating in the biological sample is prevented.

In a particularly preferred manner, said at least one transducer is mounted on the face of said sheet-like support opposite the support surface for said biological sample to apply said ultrasound towards said support surface. A more homogenous ultrasonic field is thereby obtained, even further favoring the combined effect of cold conditions and ultrasound on the biological sample. It may be appropriate for the sheet-like support to be a tray and for such tray to allow containing a fluid, such as water, to improve transmission of the applied ultrasound. The function of the fluid is on one hand to act as a coolant, and to simultaneously favor transmitting ultrasound to the biological sample.

In a particularly preferred manner, said sheet-like support is made of metal in order to increase the rigidity thereof and prevent damping to the greatest extent, and to achieve more direct transmission of the ultrasound on the biological sample.

The closable auxiliary container contains a preservation solution. Again, it has been verified that the combination of cold conditions and ultrasound plus immersion of the biological sample in a preservation solution provides better results than the sum of their individual effects as regards reducing damage to the biological sample.

In an embodiment of the device according to the invention, the sheet-like support is a tray adapted for containing a fluid.

In another preferred embodiment, the sheet like support is a tray containing water.

In another embodiment the auxiliary container comprises a false bottom located away from the base of said auxiliary container, and said false bottom being in fluid communication with the rest of said auxiliary container.

Also in a particularly preferred manner, the auxiliary container can incorporate a false bottom intended for keeping the biological sample away from the support surface of the container, said false bottom being in fluid communication with the rest of the auxiliary container. As a result, the sample can be placed in the auxiliary container as if it were floating in the preservation solution. The sample therefore does not receive such a direct action of the ultrasound and can work at higher intensities.

Furthermore, the invention also considers a method for transport and preservation of an *ex vivo* transplantable biological sample that comprises the steps of removing and washing blood out of the biological sample, placing the biological sample in a chamber delimitated by walls made of a thermal insulating material without external oxygen delivery and irradiating the sample with ultrasound.

The biological sample is kept immersed in a preservation solution without external oxygen delivery by placing said biological in a chamber delimitated by walls made of a thermal insulating material.

The biological sample is placed immersed in a closable auxiliary container, containing said preservation solution, and said auxiliary container is placed in said chamber.

In one embodiment of the method, in the irradiation step for irradiating said biological sample the ultrasound has frequencies comprised between 25 kHz and 1 MHz and a sound intensity comprised between 0.01 and 2 W/cm², and particularly preferably between 0.02 and 1 W/cm², and particularly preferably between 0.02 and 0.1 W/cm².

In another embodiment, the method comprises a cooling step for cooling the temperature in the chamber to a temperature between 0 and 15ºC, and more preferably between 2 and 10ºC, and in a particularly preferred manner from 2 to 6ºC.

The method further comprises a step consisting of keeping said biological sample immersed in a preservation solution.

Finally the method further comprises a step of placing said auxiliary container on a sheet-like support that is a tray adapted for containing a fluid and said tray being able to vibrate freely when said ultrasound is applied.

The invention also refers to the use of a device for transport and preservation of an *ex vivo* biological sample. With the device, the biological sample is maintained immersed in a preservation solution without oxygen delivery under hypothermal conditions and said sample is irradiated with ultrasound such that the viability, functionality and interaction between the different cells of said biological sample are preserved for said biological sample to be used in subsequent laboratory research. Furthermore, the invention also covers other detail features illustrated in the detailed description of an embodiment of the invention and in the attached drawings.

### Brief description of the drawings

Further advantages and features of the invention will become apparent from the following description, in which, without any limiting character, preferred embodiments of the invention are disclosed, with reference to the accompanying drawings in which:
Figure 1 shows an exploded perspective view of a first embodiment of a device for transport of an *ex vivo* biological sample.
Figure 2 shows a longitudinally sectioned front view of the device of Figure 4.
Figure 3 shows a top plan view of a first embodiment of the support tray for the biological sample of the device of Figure 1.
Figure 4 shows a side view of the tray of Figure 3.
Figure 5 shows a second embodiment of the support tray for the biological sample.
Figure 6 shows a second embodiment of the device for transport according to the invention.
Figure 7A shows a third embodiment of the device for transport according to the invention.
Figure 7B shows an alternative embodiment of the auxiliary container of the device of Figure 7A.
Figures 8A to 13 show percentage of protection of biological samples of liver and kidney with respect to conditions in the state of the art.

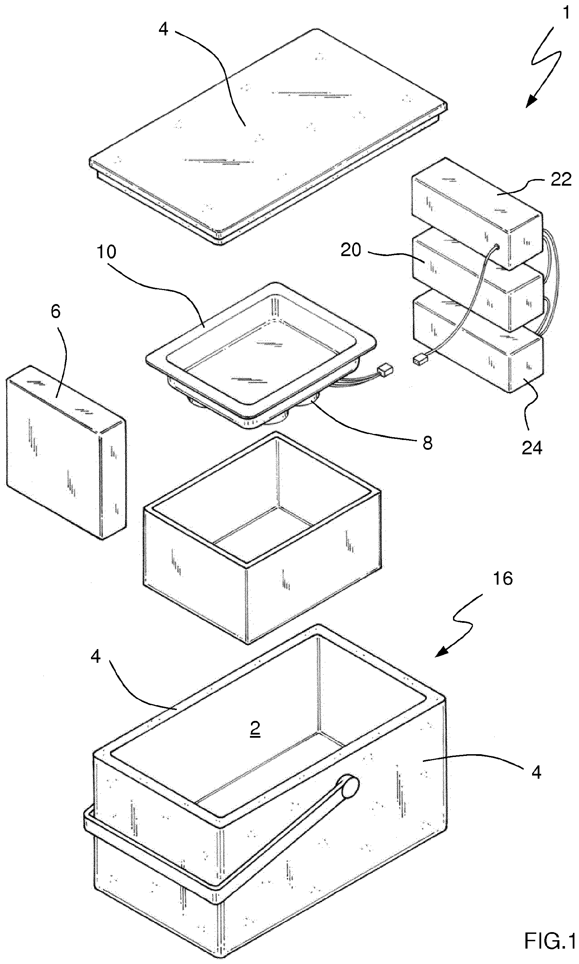

The embodiments illustrated in figures 1-6 are not encompassed by the wording of the claims but are considered as useful for understanding the invention.

### Detailed description of embodiments of the invention

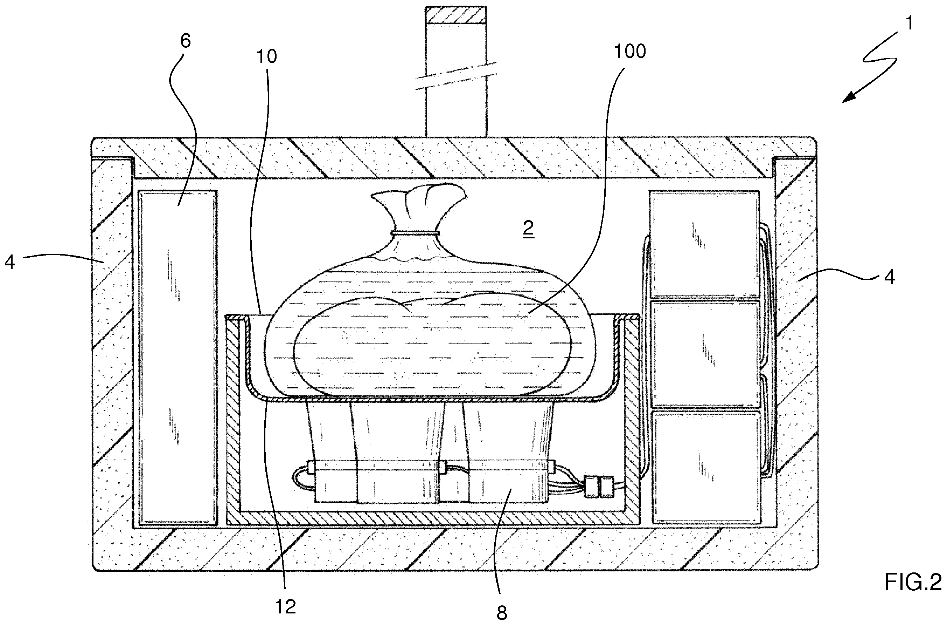

Figures 1 and 2 show a first embodiment of the device 1 for transport and preservation of an *ex vivo* transplantable biological sample 100. In a particularly preferred manner, the device a portable cooler.

As has already been previously indicated, the biological sample 100 comprises both organs that can be transplanted between donor and recipient, which can be human or animal, and tissues. Furthermore, the biological sample can also be intended for research without necessarily having to be transplanted.

The device 1 for transport and preservation has a parallelepiped-shaped main isothermal container 16 open on the upper face. The walls 4 made of a thermal insulating material of the main container 16, including its upper lid, delimitate a chamber 2 intended for containing the biological sample 100.

Cooling means 6 suitable for keeping the temperature inside the chamber 2 below the temperature outside said device 1 are provided inside the main container 16. These cooling means 6 can comprise solutions as different as ice blocks, cooling gel packs or more complex solutions comprising a compressor and a heat exchanger. The device 1 could also consist of a transportable cooler with or without an external power supply. Nevertheless, it is desirable for the solution to be as light as possible in order to not affect transportability of the assembly. The cooling means 6 allow keeping the temperature inside the chamber 2 between 0 and 15ºC. In another preferred embodiment, the temperature inside the chamber 2 is kept between 2 and 10ºC, and particularly preferably between 2 and 6ºC.

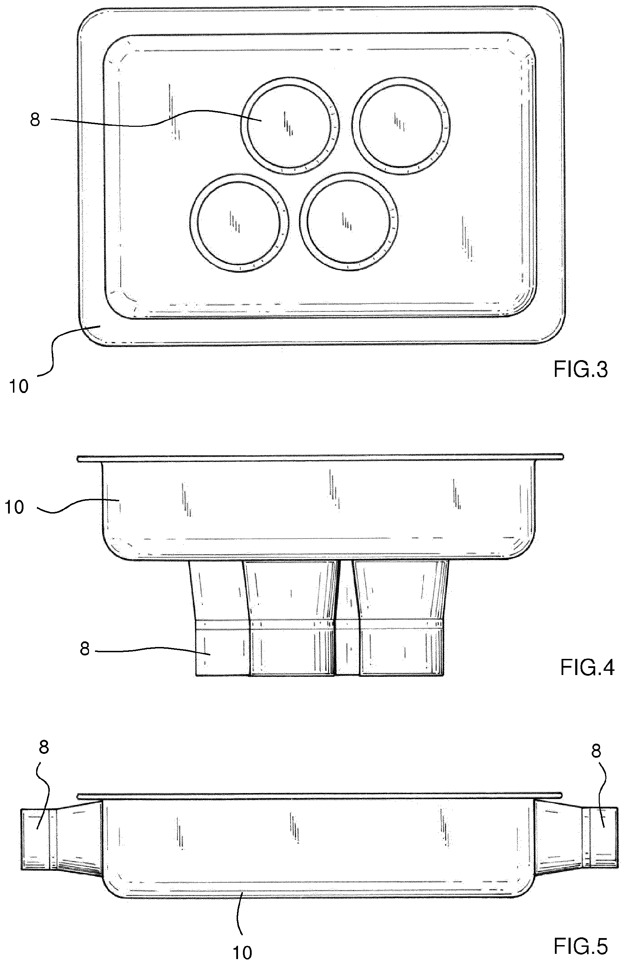

The device 1 comprises in the central part a sheet-like support 10 by way of a metal tray supported inside the chamber 2 such that it can vibrate freely. The tray of this first embodiment can be seen in detail in Figures 3 and 4. An aluminum tray with less than 1 mm, and preferably 0.5 mm thick, is used in the embodiment.

Furthermore, the device comprises an ultrasound system suitable for generating and applying ultrasound on the biological sample 100. To that end, the ultrasound system according to the drawings has an electric signal generator 20, an amplifier 22, a battery 24, and in this case four piezoelectric transducers 8.

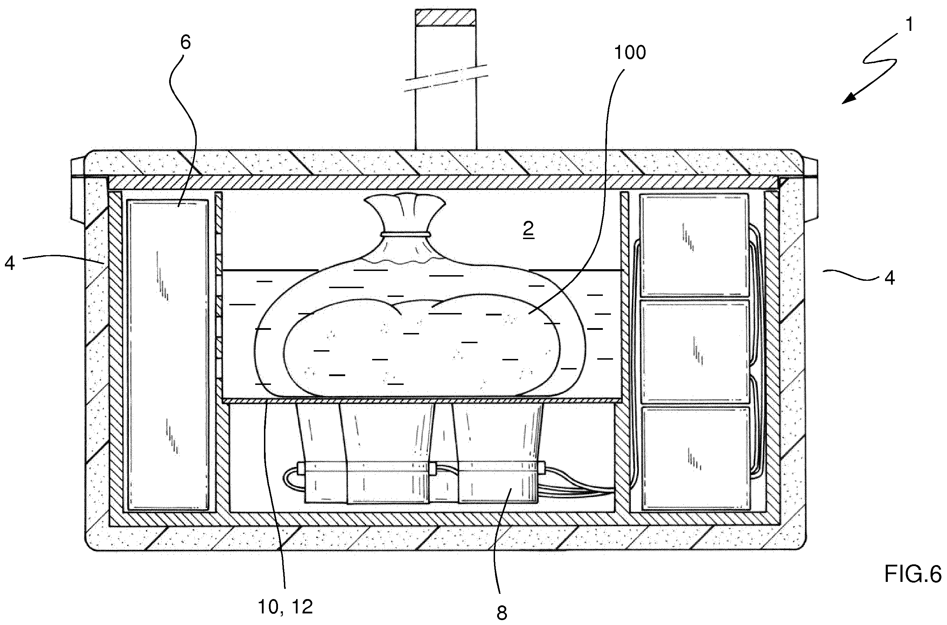

Vibrations are applied to the sheet-like support 10 by way of an aluminum tray through the four transducers 8. Said transducers 8 are mounted on the lower face of the tray. As a result of this configuration, transmission between the transducers and the biological sample 100 is more direct, because vibrations are generated directly below the lower part of the biological sample. In a particularly preferred manner, as can be seen in Figure 6, the tray can additionally contain water, but to improve transmission of ultrasound, it is envisaged that the biological sample 100 is contained in a bag or container filled with preservation solution. The preservation solutions contemplated herein are, for example, Lactated Ringer's solution, Celsior solution or University of Wisconsin solution.

In an alternative embodiment shown in Figure 5, the tray can have transducers 8 on the side walls to generate a lateral vibrational field. The transducers 8 could also alternatively be located both on side walls and on the bottom of the tray.

The ultrasound transducers 8 transmit mechanical waves having a frequency comprised between 25 kHz and 1 MHz and a sound intensity comprised between 0.1 and 2 W/cm² into said chamber 2 during transport of said biological sample 100. The intensity can preferably be comprised between 0.02 and 1 W/cm², and still more preferably between 0.02 and 0.1 W/cm². The higher the sound intensity used, the further away the transducers 8 will be and/or the larger the amount of preservation fluid or coolant will be placed in the device 1 to meet the objective of preventing local heating in the biological sample 100. Furthermore, the ultrasound can be applied continuously. Alternatively, the ultrasound can also be applied intermittently, i.e., it is not applied during the entire time transport lasts. Alternatively, it can also be applied in a pulsed manner and/or at different frequencies, either continuously or intermittently.

In a particularly preferred manner, the biological sample 100 is contained in a receptacle containing a preservation solution, which improves the results of applying ultrasound.

Other embodiments of the device 1 for transport which share many of the same features described in the preceding paragraphs are shown below. Accordingly, only those elements that are different between such embodiments will be described hereinafter, whereas for common elements reference is made to the description of the first embodiment.

The embodiment of the device 1 of Figure 6 differs primarily in that the sheet-like support 10 is integrated directly in the walls 4 of the main container 16.

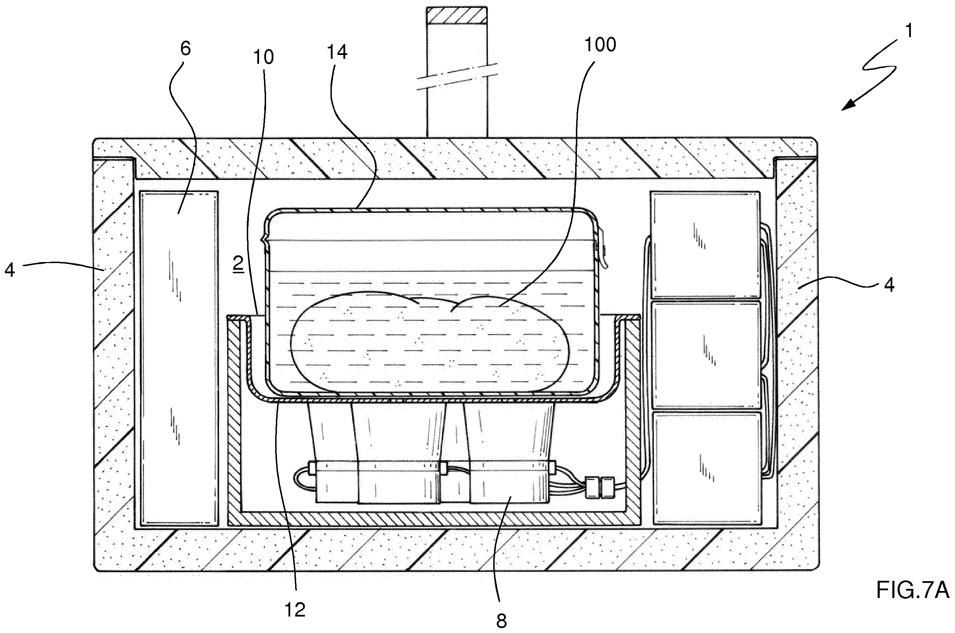

Finally, in the embodiment of the device in Figure 7A, an auxiliary container 14 is provided inside the main container 16 with a preservation solution. In a particularly preferred manner, the auxiliary container 14 is closable, with rigid walls and a lid hinged on one of its walls. In a particularly preferred manner, when the transducers are located in the lower part of the tray, the auxiliary container 14 has dimensions intended for occupying the entire emission surface of the transducers 8. As a result of the auxiliary container 14 being filled with preservation solution, the ultrasound reaches the biological sample 100 in a more efficient manner. In a particularly preferred manner, the auxiliary container 14 is manufactured by thermoforming using a formable plastic, such as for example high density polyethylene, polypropylene, polyethylene terephthalate or the like. Also in a particularly preferred manner, the material will be transparent to enable seeing the biological sample 100 without needing to open the auxiliary container 14.

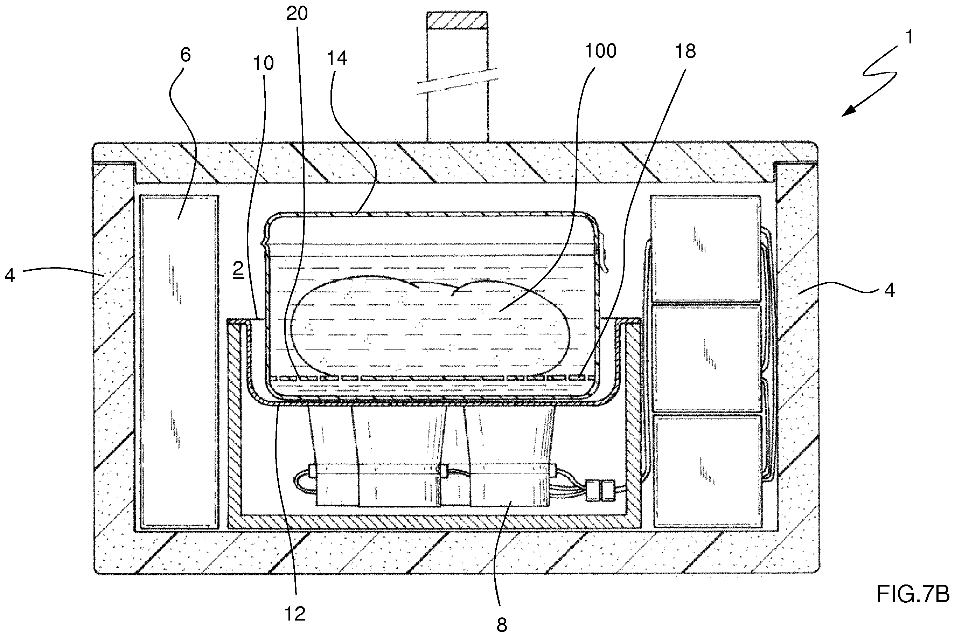

Figure 7B shows an alternative embodiment of the auxiliary container 14. In this case, the auxiliary container 14 comprises a false bottom 18 located away from the base of said auxiliary container 14, and said false bottom 18 being in fluid communication with the rest of said auxiliary container 14. The false bottom thereby places the biological sample 100 away from the support surface of the auxiliary container 14. In this case, the false bottom 18 consists of a sheet of rigid plastic supported on the side walls of the auxiliary container 14. Then to achieve proper fluid communication, a plurality of openings 20 that allow the passage of the preservation solution is provided in this case, such that said solution receives the direct effect of the ultrasound coming from the lower transducers 8.

Finally, in a particularly preferred manner, the auxiliary container 14 contains a preservation fluid under sterile conditions from the group consisting of Lactated Ringer's preservation solution, Celsior preservation solution or University of Wisconsin preservation solution. This allows a much more hygienic handling of the biological sample under more suitable conditions for the proper preservation thereof. The embodiments described up until now represent non-limiting examples, such that the person skilled in the art will understand that beyond the examples that are shown, a number of combinations of the claimed features are possible within the scope of the invention as defined by the appended claims.

### Experimental examples

Different tests conducted to put the method according to the invention into practice are described below.

### Methodology

The experimental protocol was carried out from livers and kidneys of Landrace pigs. The kidney and liver were perfused with preservation solution at 4ºC to remove blood contained in the organ, and the organ was kept under cold conditions, bathed in ice until the organs were immersed in different preservation solutions. Next the biological samples were placed in the cooler and kept in the cooler with or without ultrasound for 8 hours (liver) and 24 hours (kidney). All the procedures were performed under anesthesia and the study respected the European Union regulations concerning experiments using animals (Directive 86/609/EEC).

### Experimental design

### Protocol 1

The experimental groups formed were the following:
**GROUP 1:** cold ischemia group in the conventional transport system and with preservation solution. This group was divided into different subgroups depending on the preservation solution used.
   1.1) Perfusion of liver grafts (n=10) and kidney grafts (n=10) in Lactated Ringer's solution at 4ºC and preservation of such organs with Lactated Ringer's solution in the conventional transport system for 8 and 24 hours for liver and kidney, respectively, between 2-6ºC;
   1.2). The same as 1.1 but using Celsior solution for washing and preserving the organ;
   1.3) The same as 1.1 but using UW (University of Wisconsin) solution for washing and preserving the organ.
**GROUP 2:** cold ischemia group in the system with ultrasound (25 kHz and 0.04 W/cm²) and without preservation solution: Perfusion of liver grafts (n=10) and kidney grafts (n=10) in Lactated Ringer's solution at 4ºC to wash the organs for the purpose of removing blood contained therein. After washing, the organs were stored for 8 and 24 hours for liver and kidney, respectively, and without preservation solution in the transport system with ultrasound (25 kHz and 0.04 W/cm²) between 2-6ºC.
**GROUP 3:** cold ischemia group in the transport system with ultrasound (25 kHz and 0.04 W/cm²) and with preservation solution. The group was divided into different subgroups depending on the preservation solution used.
   3.1) Perfusion of liver grafts (n=10) and kidney grafts (n=10) in Lactated Ringer's solution at 4ºC. The organs were then stored with Lactated Ringer's solution for 8 and 24 hours for liver and kidney, respectively, in the transport system with ultrasound at a temperature comprised between 2-6ºC;
   3.2). The same as 3.1 but using Celsior solution for washing and preserving the organ;
   3..3) The same as 3.1 but using UW solution for washing and preserving the organ.

Experiments were also performed to evaluate if other frequencies and/or intensities could provide more protection than what was achieved with frequencies of 25 kHz and an intensity of 0.04 W/cm². To do this, the following experiments were performed.
**GROUP 4:** cold ischemia group in the system with ultrasound (40, 80, 200, 580 kHz and 1 MHz) and intensity of 0.04 W/cm² and with preservation solution: The group was divided into different subgroups depending on the frequency used.
   4.1) Perfusion of liver grafts (n=10) and kidney grafts (n=10) in UW solution at 4ºC The organs were then stored in UW solution for 8 and 24 hours for liver and kidney, respectively, in the transport system with ultrasound at 40 kHz and intensity of 0.04 W/cm² and between 2-6ºC;
   4.2). The same as 4.1 but using 80 kHz and intensity of 0.04 W/cm² and between 2-6ºC;
   4.3) The same as 4.1 but using 200 kHz and intensity of 0.04 W/cm² and between 2-6ºC;
   4.4) The same as 4.1 but using 580 kHz and intensity of 0.04 W/cm² and between 2-6ºC;
   4.5) The same as 4.1 but using 1 MHz and intensity of 0.04 W/cm² and between 2-6ºC.

Experiments were also performed to verify the effect of ultrasound at an intensity greater than 0.04. The following experiments were performed for that purpose:
**GROUP 5:** cold ischemia group in the system with ultrasound (25 kHz and intensity of 0.1 W/cm² and with preservation solution). Perfusion of liver grafts (n=10) and kidney grafts (n=10) in UW solution at 4ºC. The organs were then stored for 8 and 24 hours for liver and kidney, respectively, in the transport system with ultrasound at 25 kHz and intensity of 0.1 W/cm² and at a temperature comprised between 2-6ºC.

The following experimental groups were further added to evaluate the effect of the preservation solution and the cold conditions:
**GROUP 6:** cold ischemia group in the conventional transport system but without preservation solution. Perfusion of liver grafts (n=10) and kidney grafts (n=10) with Lactated Ringer's solution at 4ºC to wash the organs for the purpose of removing blood contained in the organs. After washing, the organs were stored without preservation solution in the conventional cooler (without ultrasound) for 8 and 24 hours for liver and kidney, respectively, between 2-6°C.
**GROUP 7:** non-cold condition ischemia group and combined with ultrasound (25 kHz and intensity of 0.04 W/cm²) or not. The group was divided into two subgroups.
   7.1) Perfusion of liver grafts (n=10) and kidney grafts (n=10) in UW solution at a temperature between 20-25ºC. The organs were then stored for 8 and 24 hours for liver and kidney, respectively, at a temperature comprised between 20-25°C;
   7.2) Perfusion of liver grafts (n=10) and kidney grafts (n=10) in UW solution at a temperature comprised between 20-25ºC. The organs were then stored for 8 and 24 hours for liver and kidney, respectively, at a temperature comprised between 20-25°C and with ultrasound at 25 kHz and intensity of 0.04 W/cm².

### Collecting and processing samples

At the end of ischemia, and in all the experimental groups and subgroups, liver and kidney perfusate samples were collected to assess the liver and kidney damage induced by ischemia using widely standardized techniques. Liver damage was evaluated by determining transaminase levels in the perfusate and by means of determining the caspase 3 activity in liver tissue. Kidney damage was evaluated by means of determining lactate dehydrogenase in the perfusate and caspase 3 activity in kidney tissue. MDA (malondialdehyde) levels were determined in liver and kidney tissue samples as an oxidative stress index, and ATP (adenosine triphosphate) levels were determined as an organ energy metabolism preservation index (Salahudeen AK et al., Am J Transpl 2003; 3:273-280; Omar R et al., Gut 1989;30:510-514; Peralta et al., Am J Physiol.2000; 279:G163-71).

The statistical study was conducted by means of an analysis of variance (ANOVA), and the level of statistical significance was then determined with a Student-Newman-Kels test.

The results obtained and shown schematically in the drawings are explained below in detail.

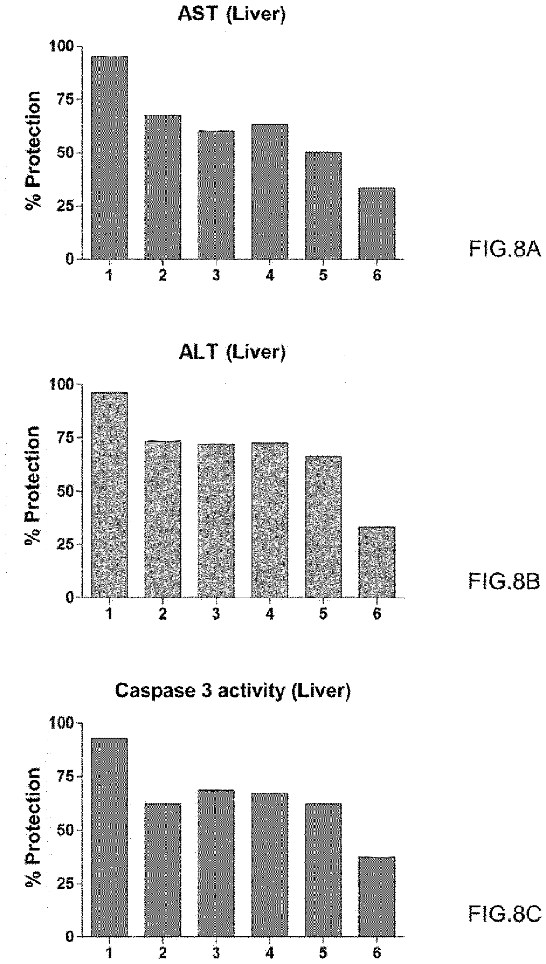

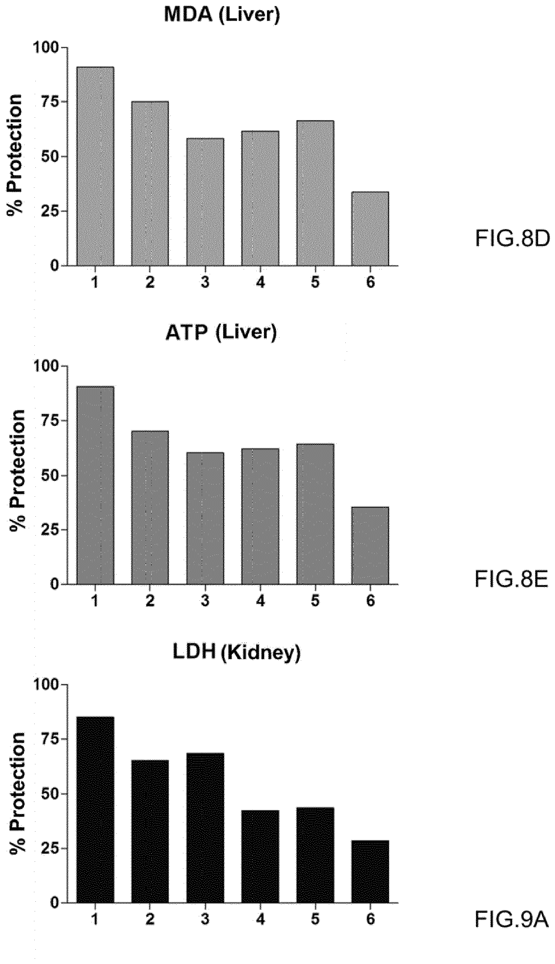

**Figures 8A to 8E****:** show the percentage of protection, or in other words, the percentage of reduction of liver injury in biological samples consisting of liver grafts under conditions 1 to 6 described below versus injury induced by the following condition: UW solution + cold conditions (2-6ºC) when the parameters indicative of liver damage, namely, AST (aspartate aminotransferase), ALT (alanine aminotransferase), caspase 3 activity, MDA (malondialdehyde) and ATP (adenosine triphosphate), were evaluated at the end of the 8 hours of cold ischemia.

### Conditions (1-6):

1: UW solution, ultrasound frequency of 25 kHz and cold conditions (2-6ºC).
2: UW solution, ultrasound frequency of 40 kHz and cold conditions (2-6ºC).
3: UW solution, ultrasound frequency of 80 kHz and cold conditions (2-6ºC).
4: UW solution, ultrasound frequency of 200 kHz and cold conditions (2-6ºC).
5: UW solution, ultrasound frequency of 580 kHz and cold conditions (2-6ºC).
6: UW solution, ultrasound frequency of 1 MHz and cold conditions (2-6ºC). An ultrasound intensity of 0.04 W/cm² was applied in all of cases 1 to 6.

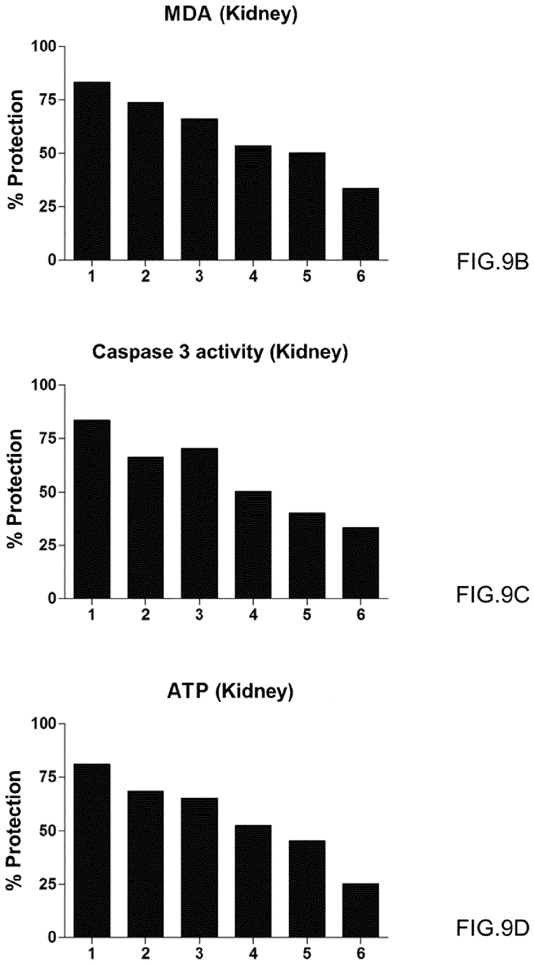

**Figures 9A to 9D****:** show the percentage of protection in biological samples consisting of kidney grafts in conditions 1 to 6 described below versus injury induced by the following condition: UW solution + cold conditions (2-6ºC) when the parameters indicative of kidney damage, namely, LDH (lactate dehydrogenase), caspase 3 activity, MDA and ATP, were evaluated at the end of the 24 hours of cold ischemia.
1 : UW solution, ultrasound frequency of 25 kHz and cold conditions (2-6ºC).
2: UW solution, ultrasound frequency of 40 kHz and cold conditions (2-6ºC).
3: UW solution, ultrasound frequency of 80 kHz and cold conditions (2-6ºC).
4: UW solution, ultrasound frequency of 200 kHz and cold conditions (2-6ºC).
5: UW solution, ultrasound frequency of 580 kHz and cold conditions (2-6ºC).
6: UW solution, ultrasound frequency of 1 MHz and cold conditions (2-6ºC).

An ultrasound intensity of 0.04 W/cm² was applied in all of cases 1 to 6.

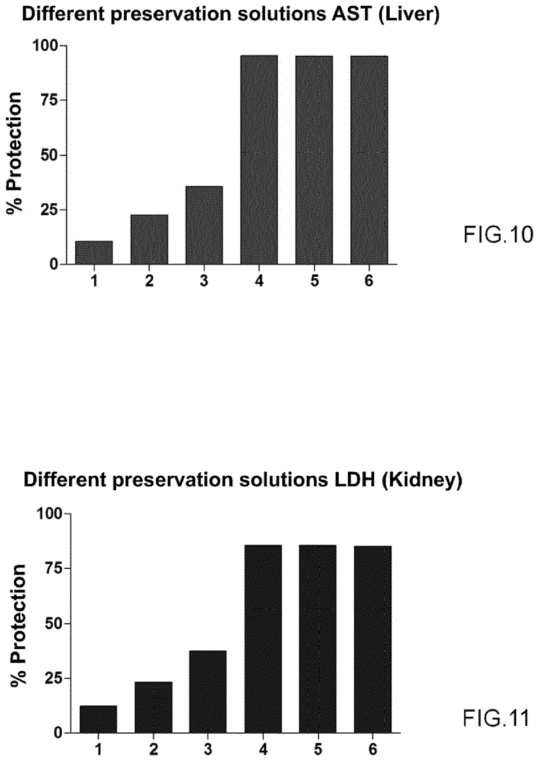

**Figure 10****:** shows the percentage of protection in liver versus injury induced by the following condition: non use of preservation solution + cold conditions (2-6ºC) + no ultrasound when the parameter indicative of liver damage, AST, was evaluated at the end of the 8 hours of cold ischemia.
1: Lactated Ringer's solution, without ultrasound and with cold conditions (2-6ºC).
2: Celsior solution, without ultrasound and with cold conditions (2-6ºC).
3: UW solution, without ultrasound and with cold conditions (2-6ºC).
4: Lactated Ringer's solution, with ultrasound (25 KHz, 0.04 W/cm²) and with cold conditions (2-6ºC).
5: Celsior solution, with ultrasound (25 KHz, 0.04 W/cm²) and with cold conditions (2-6ºC).
6: UW solution, with ultrasound (25 KHz, 0.04 W/cm²) and with cold conditions (2-6ºC).

Note: out of the preservation solutions used, the standard solution in clinical practice is UW (University of Wisconsin) preservation solution. Lactated Ringer's solution does not contain drugs and contains only mineral salts. Celsior solution contains glutathione, and UW solution contains more drugs, such as adenosine, glutathione and allopurinol.

**Figure 11****:** shows the percentage of protection in kidney versus injury induced by the following condition: non use of preservation solution + cold conditions (2-6ºC) + no ultrasound when the parameter indicative of kidney damage, LDH, was evaluated at the end of the 24 hours of cold ischemia.
1: Lactated Ringer's solution, without ultrasound and with cold conditions (2-6ºC).
2: Celsior solution, without ultrasound and with cold conditions (2-6ºC).
3: UW solution, without ultrasound and with cold conditions (2-6°C).
4: Lactated Ringer's solution, with ultrasound (25 KHz, 0.04 W/cm²) and with cold conditions (2-6°C).
5: Celsior solution, with ultrasound (25 KHz, 0.04 W/cm²) and with cold conditions (2-6°C).
6: UW solution, with ultrasound (25 KHz, 0.04 W/cm²) and with cold conditions (2-6°C).

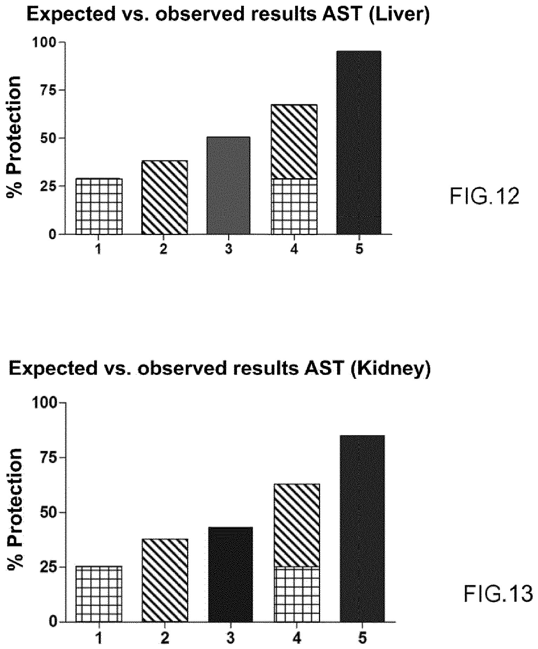

**Figure 12****:** shows the percentage of protection in liver versus injury induced by the following condition: UW preservation solution + without cold conditions (20-25°C) + no ultrasound when the parameter indicative of liver damage, AST, is evaluated at the end of the 8 hours of cold ischemia.
1: UW solution without ultrasound and with cold conditions (2-6ºC).
2: UW solution with ultrasound and without cold conditions (20-25°C).
3: Without preservation solution, with ultrasound and with cold conditions (2-6°C).
4: UW solution, with ultrasound (25 kHz, 0.04 W/cm²) and with cold conditions (2-6°C). Expected result: Protection 1 + Protection 2= Protection (1+2)
5: UW solution, with ultrasound (25 kHz, 0.04 W/cm²) and with cold conditions (2-6°C). Result actually observed. Protection 1 + Protection 2 < Protection (1+2).

**Figure 13****:** shows the percentage of protection in kidney versus injury induced by the following condition: UW preservation solution + without cold conditions (20-25°C) + no ultrasound when the parameter indicative of kidney damage, LDH, is evaluated at the end of the 24 hours of cold ischemia.
1: UW solution without ultrasound and with cold conditions (2-6ºC).
2: UW solution with ultrasound and without cold conditions (20-25°C).
3: Without preservation solution, with ultrasound and with cold conditions (2-6°C).
4: UW solution, with ultrasound (25 kHz, 0.04 W/cm²) and with cold conditions (2-6°C). Expected result: Protection 1+ Protection 2 = Protection (1+2).
5: UW solution, with ultrasound (25 kHz, 0.04 W/cm²) and with cold conditions (2-6°C). Result actually observed. Protection 1+ Protection 2 < Protection (1+2).

### Discussion of the experimental results

It is important to point out that as was expected, without applying ultrasound the temperature ranged between 2-6°C inside the cooler, in the preservation fluid and in the organ. In addition, applying ultrasound under cold conditions (2-6ºC) did not modify the temperature inside the cooler, which was between 2-6°C for all the experiments, but organ and preservation solution cooling was lost.

When applying ultrasound, the temperature ranged between 2-6°C inside the cooler, the temperature of the preservation fluid ranged between 14-16°C and the temperature of the organ between 16-18°C.

The effect of heating caused by the ultrasound is an expected result according to background documents of interest. Nevertheless, contrary to what would be expected, taking into account the vital importance of cooling the organ (4°C) and keeping the temperature of the preservation fluid and the organ between 2-6°C to store organs under hypothermal ischemia conditions, the results indicate that applying ultrasound does not negatively affect the effect of cooling the biological sample, and accordingly does not damage it.

Furthermore, however, it has surprisingly been verified that a very significant improvement is achieved, so much so that a synergistic effect between applying cold conditions inside the chamber of the device and applying ultrasound could be confirmed.

**Figures 8A to 8E****:** as shown in these drawings, under all conditions (at different frequencies and the same intensity of 0.04 W/cm²), applying ultrasound protects the liver graft under cold ischemia conditions, said protective effects being more evident under condition 1, i.e., at a frequency of 25 kHz. Other results not shown in the drawings indicate that at higher intensities, as is the case of 0.1 W/cm², protection of the liver graft is also obtained. For example, a percentage of protection or a reduction of injury of 55% is obtained at frequencies of 25 kHz and an intensity of 0.1 W/cm² versus the condition: UW solution + cold conditions (2-6ºC), when the parameter for liver damage, AST, is evaluated at the end of the 8 hours of cold ischemia.

**Figures 9A to 9D****:** show the same pattern of kidney protection as that shown in Figures 8A to 8E for the liver.

**Figure 10****:** as was expected, protection of the liver provided by the preservation solutions without applying ultrasound (conditions 1 to 3) is observed; protection of the liver graft is better if the UW preservation solution is used with respect to both solutions (Ringer or Celsior), and protection obtained by the Celsior solution is better than that obtained by Lactated Ringer's solution. In addition, when observing protection provided by the preservation solutions in the presence of ultrasound (conditions 4-6), unexpected results are obtained, indicating better protection of the ultrasound, but such protection is similar under all conditions (4-6). In other words, the same degree of protection is obtained regardless of the preservation solution used, whether it is Lactated Ringer's solution, Celsior solution or UW solution.

**Figure 11****:** shows the same pattern of kidney protection as that shown in Figure 10 for the liver.

**Figure 12****:** as was expected, cold conditions without applying ultrasound (condition 1) and using the UW solution increases protection of the liver graft by about 30% when compared with preservation with UW at 4ºC and at room temperature (20-25°C). Unexpectedly, the protection obtained by condition 2 (UW solution, in the presence of ultrasound and at room temperature) is better than that of condition 1 (UW solution and cold without ultrasound). In other words, in the presence of preservation solution at 4ºC, it is better to apply ultrasound in a chamber at room temperature than under hypothermal conditions (chamber at a temperature between 2-6ºC) and without ultrasound. The results indicating that protection obtained under condition 3 (without preservation solution, under cold conditions and with ultrasound) is better than that obtained under conditions 1 and 2 are also unexpected. These results indicate that it is better to combine ultrasound and cold conditions (even without the presence of a preservation solution) than to combine the presence of a preservation solution with cold conditions (condition 1) or with ultrasound (condition 2). Conditions 4 and 5 show the expected and observed protection, respectively, when using UW solution, ultrasound and cold conditions. The expected protection would be, in the best case, the sum of protection obtained in condition 1 and of protection obtained in condition 2. In other words, due to the heat effect induced by ultrasound, protection 1 + 2 would not be expected to correspond to the sum of conditions 1 and 2 considered separately. However, these were not the results observed. The obtained results indicated a synergistic effect when both treatments (cold conditions and ultrasound) are combined because the protection obtained when both treatments are combined is much better than the sum of protections obtained when both treatments are applied separately. Furthermore, the protection obtained when both treatments are combined and in the presence of preservation solution is much better than that obtained when both treatments are combined without preservation solution (condition 3).

**Figure 13****:** shows the same pattern of kidney protection as that shown in Figure 5 for the liver.

Accordingly, taking all the results into account, the combination of preservation solution, ultrasound and cold conditions can be an extremely efficient strategy for transport and preservation of organs during cold ischemia.

A method and equipment for transporting and storing biological samples under hypothermal conditions and without oxygen delivery under better conditions than those currently available, and with effective transmission of ultrasound to the organ or tissue inside the chamber, are provided. All this allows reducing the harmful effects of cold ischemia and increasing viability of grafts before they are implanted in the recipient, thereby preventing having to do another transplant.

The equipment and method for preservation could also be useful in secondary organs; the number of organs available for transplant could accordingly increase, thereby reducing waiting lists. Furthermore, since the injuries induced by cold ischemia during the conservation and transport of organs is reduced, the time during which organs are transported in the cooler until they are implanted in the recipient can be extended. Furthermore, the equipment is easy to transport in order to prevent, among other factors, logistic complications resulting from dynamic preservation of the organ.

## Claims

1. A device (1) for transport and preservation of an ex *vivo* biological sample (100) for subsequent transplant in a living human being or animal, comprising a chamber (2) for containing said biological sample (100), delimitated by walls (4) made of a thermal insulating material, cooling means (6) for keeping the temperature inside said chamber (2) below the temperature outside said device (1), and at least an ultrasound system suitable for generating and applying ultrasound on said biological sample (100), wherein the device further comprises a closable auxiliary container (14) for containing a preservation solution, without external oxygen delivery and for containing said biological sample (100) immersed in said preservation solution, **characterized in that** the device further comprises a sheet-like support (10) in said chamber (2) suitable for supporting said closable auxiliary container (14) containing said biological sample (100), said sheet-like support (10) being able to vibrate freely when said ultrasound is applied, and wherein said ultrasound system comprises at least one transducer (8) mounted on at least one of the walls of said sheet-like support (10).

2. The device (1) according to claim 1, wherein said ultrasound system is suitable for emitting said ultrasound at a frequency comprised between 25 kHz and 1 MHz and a sound intensity comprised between 0.01 and 2 W/cm².

3. The device (1) according to claim 2, wherein said sound intensity is comprised between 0.02 and 1 W/cm², and preferably between 0.02 and 0.1 W/cm²

4. The device (1) according to any of claims 1 to 3, wherein said cooling means (6) are suitable for keeping the temperature inside said chamber (2) between 0 and 15°C, preferably suitable for keeping the temperature inside said chamber (2) between 2 and 10°C, and particularly preferably between 2 and 6°C.

5. The device (1) according to claim 1, wherein said at least one transducer (8) is mounted on the face of said sheet-like support (10), preferably made of metal, opposite the support surface (12) for said biological sample (100) to apply said ultrasound towards said support surface (12).

6. The device (1) according to any of claims 1 4- to 5, wherein sheet-like support (10) is a tray adapted for containing a fluid.

7. The device (1) according to any of claims 1 to 6, wherein said auxiliary container (14) comprises a false bottom (18) located away from the base of said auxiliary container (14), and said false bottom (18) being in fluid communication with the rest of said auxiliary container (14).

8. A method for transport of an ex *vivo* biological sample (100) for subsequent transplant in a living human being or animal, wherein said method comprises the following steps:
[a] removing blood from said biological sample (100) under cold conditions and rapidly cooling said biological sample (100),
[b] keeping said biological sample (100) immersed in a preservation solution without external oxygen delivery by placing said biological sample in a chamber (2) delimitated by walls (4) made of a thermal insulating material,
[c] irradiating said sample with ultrasound, and
wherein said biological sample (100) is placed immersed in a closable auxiliary container (14) containing said preservation solution, and said auxiliary container (14) is placed in said chamber (2), and wherein said auxiliary container (14) is placed on a sheet-like support (10) that is a tray adapted for containing a fluid and said tray being able to vibrate freely when said ultrasound is applied through at least one transducer (8) mounted on at least one of the walls of said sheet-like support (10).

9. The method according to any of claim 8, wherein in said irradiation step for irradiating said biological sample (100), the ultrasound has frequencies comprised between 25 kHz and 1 MHz and a sound intensity comprised between 0.01 and 2 W/cm².

10. The method according to claim 9, wherein said ultrasound has an intensity comprised between 0.02 and 1 W/cm², and preferably between 0.02 and 0.1 W/cm²

11. The method according to any of claims 8 to 10, wherein it further comprises a cooling step for cooling the temperature in said chamber (2) to a temperature between 0 and 15°C, wherein preferably in said cooling step the temperature in said chamber (2) is kept between 2 and 10°C and particularly preferably between 2 and 6°C.

12. Use of a device (1) for transport and preservation of an ex *vivo* biological sample (100) according to any of claims 1 to 7, wherein said biological sample (100) is maintained immersed in a preservation solution without oxygen delivery under hypothermal conditions and said sample (100) is irradiated with ultrasound such that the viability, functionality and interaction between the different cells of said biological sample (100) are preserved for said biological sample (100) to be used in subsequent laboratory research.

## Patentansprüche

1. Vorrichtung (1) zum Transport und Konservierung einer biologischen *Ex-vivo*-Probe (100) für die spätere Transplantation in einen lebenden Menschen oder in ein lebendes Tier, umfassend eine Kammer (2) zum Enthalten der genannten biologischen Probe (100), welche Kammer (2) von Wänden (4) hergestellt aus einem Wärmedämmstoff begrenzt ist, Kühlmittel (6) zum Halten der Temperatur innerhalb der genannten Kammer (2) unter der Temperatur außerhalb der genannten Vorrichtung (1) und mindestens ein Ultraschallsystem, welches zum Erzeugen von Ultraschall und zum Anwenden desselben auf die genannte biologische Probe (100) geeignet ist, wobei die Vorrichtung zusätzlich einen schließbaren Hilfsbehälter (14) zum Enthalten einer Konservierungslösung, ohne äußere Sauerstoffzufuhr und zum Enthalten der genannten biologischen Probe (100) in die genannte Konservierungslösung eingetaucht, umfasst, **dadurch gekennzeichnet, dass** die Vorrichtung zusätzlich einen folienartigen Träger (10) in der genannten Kammer (2) umfasst, welcher dafür geeignet ist, den genannten schließbaren Hilfsbehälter (14) enthaltend die genannte biologische Probe (100) zu tragen, wobei der genannte folienartige Träger (10) in der Lage ist frei zu vibrieren, wenn der genannte Ultraschall angewendet wird, und wobei das genannte Ultraschallsystem mindestens einen Wandler (8) umfasst, welcher auf mindestens einer der Wände des genannten folienartigen Trägers (10) montiert ist.

2. Vorrichtung (1) nach Anspruch 1, wobei das genannte Ultraschallsystem dafür geeignet ist, den genannten Ultraschall bei einer Frequenz, welche zwischen 25 kHz und 1 MHz liegt, und einer Schallstärke, welche zwischen 0,01 und 2 W/cm² liegt, auszugeben.

3. Vorrichtung (1) nach Anspruch 2, wobei die genannte Schallstärke zwischen 0,02 und 1 W/cm², und vorzugsweise zwischen 0,02 und 0,1 W/cm² liegt.

4. Vorrichtung (1) nach einem der Ansprüche 1 bis 3, wobei die genannten Kühlmittel (6) dafür geeignet sind, die Temperatur innerhalb der genannten Kammer (2) zwischen 0 und 15°C zu halten, vorzugsweise dafür geeignet ist, die Temperatur innerhalb der genannten Kammer (2) zwischen 2 und 10ºC, und insbesondere vorzugsweise zwischen 2 und 6°C zu halten.

5. Vorrichtung (1) nach Anspruch 1, wobei der genannte mindestens eine Wandler (8) auf der Fläche des genannten folienartigen Trägers (10), welcher vorzugsweise aus Metall hergestellt ist, der Trägeroberfläche (12) für die genannte biologische Probe (100) entgegengesetzt, montiert ist, um den genannten Ultraschall zur genannten Trägeroberfläche (12) hin anzuwenden.

6. Vorrichtung (1) nach einem der Ansprüche 1 bis 5, wobei der folienartige Träger (10) eine Schale ist, welche dazu angepasst ist, ein Fluid zu enthalten.

7. Vorrichtung (1) nach einem der Ansprüche 1 bis 6, wobei der genannte Hilfsbehälter (14) einen doppelten Boden (18) umfasst, welcher sich von der Basis des genannten Hilfsbehälters (14) entfernt befindet, und wobei der genannte doppelte Boden (18) in Fluidverbindung mit dem Rest des genannten Hilfsbehälters (14) ist.

8. Verfahren zum Transport einer biologischen *Ex-vivo*-Probe (100) für die spätere Transplantation in einen lebenden Menschen oder in ein lebendes Tier, wobei das genannte Verfahren die folgenden Schritte umfasst:
[a] das Entnehmen von Blut aus der genannten biologischen Probe (100) unter kalten Bedingungen und das schnelle Kühlen der genannten biologischen Probe (100),
[b] das Halten der genannten biologischen Probe (100) in eine Konservierungslösung eingetaucht, ohne äußere Sauerstoffzufuhr, indem die genannte biologische Probe in einer Kammer (2) platziert wird, welche von Wänden (4) hergestellt aus einem Wärmedämmstoff begrenzt ist, und
[c] das Bestrahlen der genannten Probe mit Ultraschall, und
wobei die genannte biologische Probe (100) in einen schließbaren Hilfsbehälter (14) eingetaucht, welcher die genannte Konservierungslösung enthält, platziert wird, und der genannte Hilfsbehälter (14) in die genannte Kammer (2) platziert wird, und wobei der genannte Hilfsbehälter (14) auf einem folienartigen Träger (10), welcher eine Schale ist, welche dazu angepasst ist, ein Fluid zu enthalten, platziert wird, und wobei die genannte Schale in der Lage ist frei zu vibrieren, wenn der genannte Ultraschall über mindestens einen Wandler (8) angewendet wird, welcher auf mindestens einer der Wände des genannten folienartigen Trägers (10) montiert ist.

9. Verfahren nach einem der Ansprüche 8, wobei im genannten Bestrahlungsschritt zum Bestrahlen der genannten biologischen Probe (100), der Ultraschall Frequenzen, welche zwischen 25 kHz und 1 MHz liegen, und eine Schallstärke, welche zwischen 0,01 und 2 W/cm² liegt, aufweist

10. Verfahren nach Anspruch 9, wobei der genannte Ultraschall eine Stärke aufweist, welche zwischen 0,02 und 1 W/cm², und vorzugsweise zwischen 0,02 und 0,1 W/cm² liegt.

11. Verfahren nach einem der Ansprüche 8 bis 10, wobei es zusätzlich einen Kühlschritt zum Kühlen der Temperatur in der genannten Kammer (2) auf eine Temperatur zwischen 0 und 15°C umfasst, wobei vorzugsweise im genannten Kühlschritt die Temperatur in der genannten Kammer (2) zwischen 2 und 10°C, und insbesondere vorzugsweise zwischen 2 und 6°C gehalten wird.

12. Verwendung einer Vorrichtung (1) zum Transport und Konservierung einer biologischen *Ex-vivo*-Probe (100) nach einem der Ansprüche 1 bis 7, wobei die genannte biologische Probe (100) in eine Konservierungslösung eingetaucht gehalten wird, ohne Sauerstoffzufuhr und unter hypothermischen Bedingungen, und die genannte Probe (100) mit Ultraschall bestrahlt wird, sodass die Lebensfähigkeit, Funktionalität und Wechselwirkung zwischen den unterschiedlichen Zellen der genannten biologischen Probe (100) konserviert wird, damit die genannte biologische Probe (100) in spätere Laboruntersuchung verwendet wird.

## Revendications

1. Dispositif (1) pour le transport et la conservation d'un échantillon biologique *ex vivo* (100) pour transplantation subséquente chez un être humain ou animal vivant, comprenant une chambre (2) pour contenir ledit échantillon biologique (100), délimitée par des parois (4) faites en un matériau isolant thermique, des moyens de refroidissement (6) pour maintenir la température à l'intérieur de ladite chambre (2) en dessous de la température à l'extérieur dudit dispositif (1), et au moins un système à ultrasons approprié pour générer et appliquer des ultrasons sur ledit échantillon biologique (100), dans lequel le dispositif comprend en outre un récipient auxiliaire fermable (14) pour contenir une solution de conservation, sans apport extérieur d'oxygène et pour contenir ledit échantillon biologique (100) immergé dans ladite solution de conservation, **caractérisé en ce que** le dispositif comprend en outre un support en forme de feuille (10) dans ladite chambre (2) approprié pour supporter ledit récipient auxiliaire fermable (14) contenant ledit échantillon biologique (100), ledit support en forme de feuille (10) pouvant vibrer librement lorsque lesdits ultrasons sont appliqués, et dans lequel ledit système à ultrasons comprend au moins un transducteur (8) monté sur au moins une des parois dudit support en forme de feuille (10).

2. Dispositif (1) selon la revendication 1, dans lequel ledit système à ultrasons est approprié pour émettre lesdits ultrasons à une fréquence comprise entre 25 kHz et 1 MHz et une intensité sonore comprise entre 0,01 et 2 W/cm².

3. Dispositif (1) selon la revendication 2, dans lequel ladite intensité sonore est comprise entre 0,02 et 1 W/cm², et préférablement entre 0,02 et 0,1 W/cm².

4. Dispositif (1) selon l'une quelconque des revendications 1 à 3, dans lequel lesdits moyens de refroidissement (6) sont appropriés pour maintenir la température à l'intérieur de ladite chambre (2) entre 0 et 15 °C, préférablement appropriés pour maintenir la température à l'intérieur de ladite chambre (2) entre 2 et 10 °C, et en particulier préférablement entre 2 et 6 °C.

5. Dispositif (1) selon la revendication 1, dans lequel ledit au moins un transducteur (8) est monté sur la face dudit support en forme de feuille (10), préférablement fait en métal, à l'opposé de la surface de support (12) pour ledit échantillon biologique (100) pour appliquer lesdits ultrasons vers ladite surface de support (12).

6. Dispositif (1) selon l'une quelconque des revendications 1 à 5, dans lequel le support en forme de feuille (10) est un plateau adapté pour contenir un fluide.

7. Dispositif (1) selon l'une quelconque des revendications 1 à 6, dans lequel ledit récipient auxiliaire (14) comprend un double fond (18) situé à l'écart de la base dudit récipient auxiliaire (14), et ledit double fond (18) étant en communication fluidique avec le reste dudit récipient auxiliaire (14).

8. Méthode pour le transport d'un échantillon biologique *ex vivo* (100) pour transplantation subséquente chez un être humain ou animal vivant, dans laquelle ladite méthode comprend les étapes suivantes :
[a] retirer du sang depuis ledit échantillon biologique (100) sous des conditions froides et refroidir rapidement ledit échantillon biologique (100),
[b] maintenir ledit échantillon biologique (100) immergé dans une solution de conservation sans apport extérieur d'oxygène en plaçant ledit échantillon biologique dans une chambre (2) délimitée par des parois (4) faites d'un matériau isolant thermique,
[c] irradier ledit échantillon avec des ultrasons, et
dans laquelle ledit échantillon biologique (100) est placé immergé dans un récipient auxiliaire fermable (14) contenant ladite solution de conservation, et ledit récipient auxiliaire (14) est placé dans ladite chambre (2), et dans laquelle ledit récipient auxiliaire (14) est placé sur un support en forme de feuille (10) qui est un plateau approprié pour contenir un fluide et ledit plateau pouvant vibrer librement lorsque lesdits ultrasons sont appliqués à travers au moins un transducteur (8) monté sur au moins une des parois dudit support en forme de feuille (10).

9. Méthode selon l'une quelconque de la revendication 8, dans laquelle dans ladite étape d'irradiation pour irradier ledit échantillon biologique (100), les ultrasons ont des fréquences comprises entre 25 kHz et 1 MHz et une intensité sonore comprise entre 0,01 et 2 W/cm².

10. Méthode selon la revendication 9, dans laquelle lesdits ultrasons ont une intensité comprise entre 0,02 et 1 W/cm², et préférablement entre 0,02 et 0,1 W/cm².

11. Méthode selon l'une quelconque des revendications 8 à 10, dans laquelle elle comprend en outre une étape de refroidissement pour refroidir la température dans ladite chambre (2) à une température entre 0 et 15 °C, dans laquelle préférablement dans ladite étape de refroidissement la température dans ladite chambre (2) est maintenue entre 2 et 10 °C et en particulier préférablement entre 2 et 6 °C.

12. Utilisation d'un dispositif (1) pour le transport et la conservation d'un échantillon biologique *ex vivo* (100) selon l'une quelconque des revendications 1 à 7, dans laquelle ledit échantillon biologique (100) est maintenu immergé dans une solution de conservation sans apport d'oxygène sous des conditions hypothermiques et ledit échantillon (100) est irradié avec des ultrasons de telle sorte que la viabilité, fonctionnalité et interaction entre les différentes cellules dudit échantillon biologique (100) sont préservées pour que ledit échantillon biologique (100) soit utilisé en recherche subséquente de laboratoire.
